# EUROPÄISCHE PATENTANMELDUNG

(11) **EP 2 140 886 A1**
(43) Veröffentlichungstag der Anmeldung: **06.01.2010**
(21) Anmeldenummer: 09008174.6
(22) Anmeldetag: 22.06.2009
(51) Int. Cl.: A61L 2/28, A61L 2/26, G08B 21/24, C11D 3/00

(54) **Vorrichtung zur Desinfektionskontrolle**

(30) Priorität: 26.06.2008 DE 102008030463
(71) Anmelder: Werner, Daniel, 61440 Oberursel (DE)
(72) Erfinder: Werner, Daniel, 61440 Oberursel (DE)
(74) Vertreter: Jochem, Bernd

(57) **Zusammenfassung**

Die Vorrichtung zur Desinfektionskontrolle ist an einem Ausgang eines Waschraums mit Einrichtungen zum Waschen und Desinfizieren der Hände angeordnet. Sie besteht aus einem verriegelbaren Absperrorgan 2, dessen Verriegelungseinrichtung 8 von einer Detektoreinheit 9 steuerbar ist. Die Sensoren der Detektoreinheit 9 registrieren das Vorhandensein bzw. Fehlen von Rückständen des Desinfektionsmediums oder einer darin enthaltenen Markierungssubstanz an den Händen der Personen, die den Waschraum verlassen wollen. Nur wenn an ihren Händen Rückstände des Desinfektionsmediums festgestellt werden, wird das Absperrorgan 2 entriegelt.

## Beschreibung

Die Erfindung betrifft eine Vorrichtung zur Desinfektionskontrolle.

Personen, die in potentiell gesundheitsgefährdenden Bereichen der Lebensmittelherstellung oder im Servicebereich von Hotels, Gaststätten, Krankenhäusern usw. tätig sind, müssen auf äußerste Reinlichkeit achten, um die Übertragung von Krankheitserregern zu vermeiden. Besonders die Hände dieser Personen sind, z.B. nach einem Toilettenbesuch, ein Risikopotential, denn nicht immer wird hier die nötige Sorgfalt beachtet. Oft wird sogar das Reinigen der Hände völlig unterlassen.

Auch die HACCP - Verordnung schreibt erhöhte Sorgfalt in diesen Bereichen vor.

Die vorliegende Erfindung hat sich die Aufgabe gestellt, für den genannten Personenkreis eine Vorrichtung zur Verfügung zu stellen, die diese Personen zwingt, entsprechende Sorgfalt bezüglich der Reinlichkeit vor Ort walten zu lassen.

Die Lösung dieser Aufgabe erfolgt mit den im kennzeichnenden Teil des Anspruchs 1 angegebenen Merkmalen.

Vorteilhafte Weiterbildungen und Ausgestaltungen ergeben sich aus den Unteransprüchen.

Ein Ausführungsbeispiel ist in der Zeichnung dargestellt und in der nachstehenden Beschreibung näher erläutert.

Es zeigen:

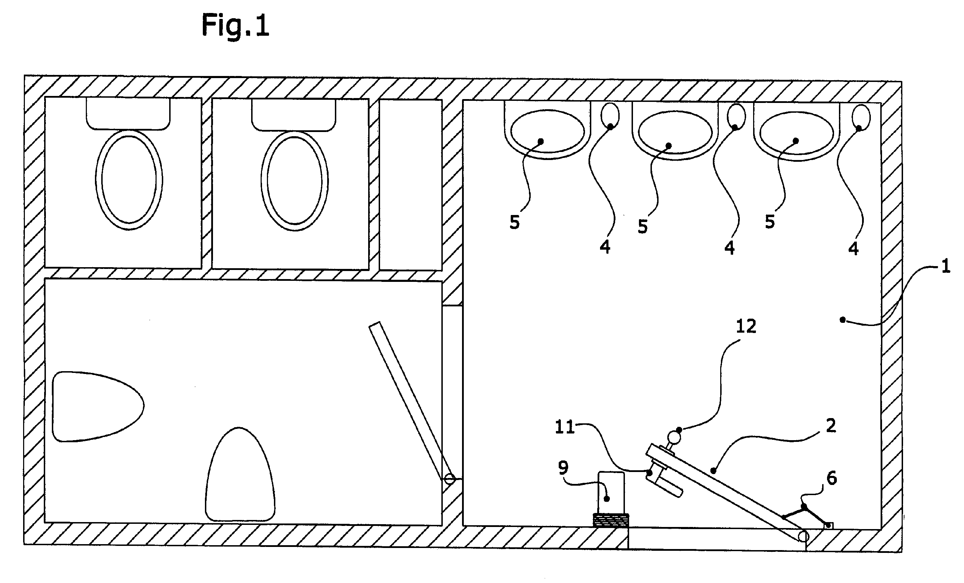

- Fig. 1: einen typischen Grundriss einer Toilettenanlage,

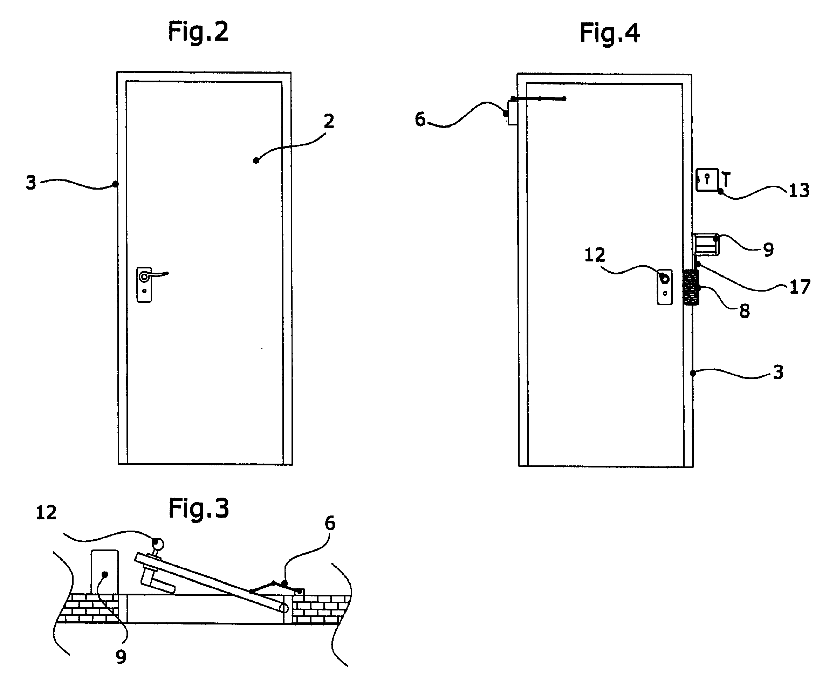

- Fig. 2: die Eingangstür der Toilettenanlage von außen gesehen,
- Fig. 3: die Tür in der Draufsicht,
- Fig. 4: die Tür von innen gesehen,
- Fig. 5: die einzelnen Baugruppen einer an der Tür angebrachten Vorrichtung zur Desinfektionskontrolle in ihrer Lage zueinander in der Seitenansicht in größerem Maßstab,
- Fig. 6: die Vorrichtung nach Fig. 5 in der Frontansicht,
- Fig. 7: die Prinzipdarstellung einer Detektoreinheit mit Distanzstiften in einer Ansicht von unten und in der Seitenansicht.

Die erfindungsgemäße Vorrichtung zur Desinfektionskontrolle besteht im Wesentlichen aus zwei Baugruppen, nämlich einem Absperrorgan, z. B. in Form einer Tür 2, mit einer steuerbaren Verriegelungseinrichtung 8 sowie einer Detektoreinheit 9 mit einem in dieser integrierten, aber hier nicht dargestellten Sensor. Diese beiden Baugruppen sind durch eine Steuerleitung 17 elektrisch verbunden.

Falls die Vorrichtung nicht erfindungsgemäß funktionieren sollte, ist ein Notfallset 13 vorgesehen, das aus einem verschlossenen Schlüsselkasten 14 mit einer Frontscheibe aus Glas besteht. Darin ist ein Schlüssel 15 zum Öffnen der Toilettentür 2 aufbewahrt, der nur zugänglich ist durch Zerstörung der Glasscheibe des Schlüsselkastens mithilfe des ebenfalls vorgesehenen Hammers 16.

Die Detektoreinheit kann einen z. B. auf Infrarot oder sichtbares Licht eines bestimmten Wellenlängenbereichs empfindlichen Sensor enthalten und in Gebrauchshöhe an oder neben dem Türrahmen 3 angebracht sein. Als Verriegelungseinrichtung 8 dient im Beispielsfall ein elektromagnetischer Riegel einer handelsüblichen Baugruppe, die am Türrahmen 3 montiert ist.

Die Detektoreinheit 9 prüft, ob die Hände vor dem Verlassen der Toilette vom Personal vorschriftsmäßig gereinigt wurden. Die Prüfung des Reinigungsgrades erfolgt, indem die Hände vor die Sensorfläche 10, z. B. an der Unterseite der Detektoreinheit 9, gehalten werden. Nur wenn der Sensor dabei bestimmte Rückstände des verwendeten Desinfektionsmediums auf den Handflächen erkennt, gibt die von ihm gesteuerte Verriegelungseinrichtung 8 die Tür zum Verlassen der Toilette frei.

Der Ablauf der Kontrolle ist nun folgender:

Der Mitarbeiter betritt die Toilettenanlage durch die Einund Ausgangstür 2. Diese wird geöffnet mittels einer normalen Türklinke 11 und ist immer von außen zugänglich. Die Tür 2 wird hinter ihm automatisch durch einen Schließer 6 geschlossen. Der Mitarbeiter benutzt die Anlage und begibt sich danach in den Waschraum 1.

Verlassen kann er die Toilettenanlage jetzt noch nicht, denn die Tür 2 ist von innen ohne gereinigte Hände nicht zu öffnen. Vielmehr befindet sich an der Innenseite neben der Tür 2 die Detektoreinheit 9. An diese muss der Mitarbeiter wenigstens eine seiner Handflächen heranführen, damit ein Sensor, ggf. in Verbindung mit einer Messeinrichtung, in der Detektoreinheit 9 feststellen kann, ob die Hände gereinigt worden sind. Die Tür 2 öffnet sich automatisch oder hat einen Zugknopf 12, mit dem sie geöffnet werden kann. Das kann aber nur geschehen, wenn die Detektoreinheit 9 desinfizierte Handflächen registriert und daraufhin den elektromechanischen Riegel 8 freigibt bzw. zurückzieht.

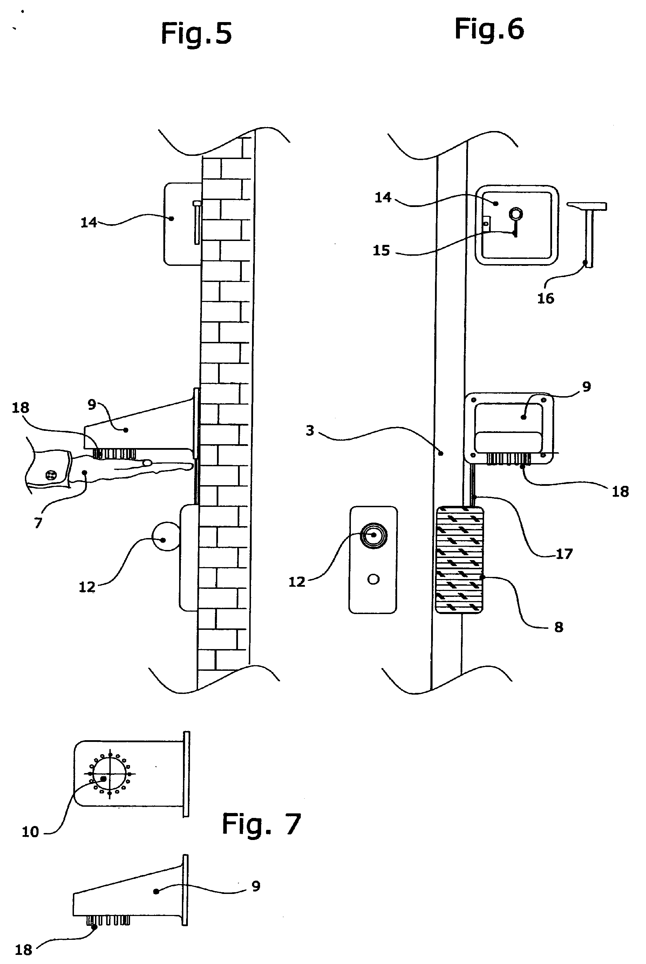

Die Sensorfläche 10 der Detektoreinheit 9 ist im Ausführungsbeispiel nach unten gerichtet und mit einzelnen oder z. B. einem Kranz von Distanzstiften 18 versehen, damit der Messabstand exakt definiert ist. Die Kontrolle der Hände geschieht demnach an der Unterseite der Detektoreinheit 9, wie in Fig.5 dargestellt.

Im Waschraum 1 befinden sich normale Waschbecken 5. Unmittelbar daneben befinden sich Spender 4, angefüllt mit einem Reinigungsfluid, das normalerweise aus einem Seifen- und Alkoholgemisch besteht, dem eine Markierungssubstanz beigemischt ist. Auf den Gehalt dieses Gemisches ist der Detektor, der sich innerhalb der Detektoreinheit 9 befindet, kalibriert.

Hat sich der Mitarbeiter vorschriftsmäßig die Hände gereinigt, so sind doch noch unvermeidbare Rückstände des Desinfektionsmittels auf den Händen vorhanden. Die Detektoreinheit 9 registriert diese und gibt daraufhin über den elektromagnetischen Riegel 8 die Tür 2 frei. Falls nicht, muss er zurück und seine Hände erneut reinigen oder, wenn es gar nicht anders geht, den Hammer benutzen.

Ein besonderer Vorteil der Erfindung ist, dass die genannten Baugruppen ohne oder mit nur geringer Änderung der Bausubstanz installiert werden können. Dies gilt unabhängig vom Funktionsprinzip des gewählten Detektors und der Art der dem Reinigungs- und Desinfektionsmittel beigemischten, für die Detektion benutzten Markierungssubstanz. Bei dieser handelt es sich vorzugsweise um einen Fluoreszenzfarbstoff, der nach Anregung durch Bestrahlung mit UV-Strahlen oder einem Licht relativ kürzerer Wellenlänge ein Licht relativ längerer Wellenlänge abstrahlt. Es ist eine Vielzahl derartiger Fluoreszenzfarbstoffe bekannt, auch solche, die für Kosmektika, Waschmittel (z. B. sog. Aufheller), Seifen, Shampoos oder sogar für Lebensmittel zugelassen sind.

Wenn der Rückstand an Fluoreszenzfarbstoff auf der gereinigten Hand durch die Strahlung z. B. einer oder mehrerer an oder in der Detektoreinheit 9 installierter Leuchtdioden, deren Emissionsmaximum auf die Absorption des Farbstoffs abgestimmt ist, zur Fluoreszenz angeregt wird, leuchtet er auf mit der ihm eigenen Emissionswellenlänge. Diese liegt vorzugsweise außerhalb des Bereichs der Augenempfindlichkeit. Mittels eines Farbfilters, das speziell diese Strahlung passieren lässt, aber Licht anderer Wellenlängen absorbiert, und eines dahinter angeordneten, die Fluoreszenzstrahlung registrierenden Sensors, kann die Detektoreinheit zwischen einer fluoreszierenden, gereinigten und einer nicht fluoreszierenden, ungereinigten Hand unterscheiden. Als Sensor kommt z. B. ein Chip einer Digitalkamera oder eine Fotozelle in Frage.

Die teilweise Abschirmung der Sensorfläche 10 gegen das Licht im Waschraum durch sie umgebende, bis auf eine Einführöffnung für die Hände geschlossene Gehäusewände der Detektoreinheit 9 kann im Einzelfall zweckmäßig sein.

Andere Markierungssubstanzen könnten z. B. die Reflexion, die Absorption, die Durchblutung, die Farbe oder die elektrischen Eigenschaften der gereinigten Handflächen bei Bestrahlung mit Licht, anderen elektromagnetischen Wellen oder Ultraschall beeinflussen oder ein auf der Haut verdunstender Stoff sein, der beispielsweise elektrische Eigenschaften von Sensoren des Detektors verändert. Denkbar sind auch Detektoren für bestimmte Gerüche, die elektrische Leitfähigkeit der Haut oder die Wärmestrahlung der Hand. Wesentlich ist dabei, dass erfindungsgemäß mit einfachen Mitteln rigoros für Einhaltung der Reinheitsvorschriften gesorgt wird, ohne Kontrollpersonen einsetzen und versehentliches Fehlverhalten einzelner Mitarbeiter in beschämender Weise bloßstellen zu müssen.

Es versteht sich, dass anstelle der beschriebenen Notfalleinrichtung 14, 15, 17 z. B. auch eine Notrufeinrichtung vorhanden sein kann. Die steuerbare Verriegelungseinrichtung 8 könnte an der Tür 2 montiert und durch eine Funkverbindung mit der Detektoreinheit 9 verbunden sein. Außerdem kann zusätzlich im äußeren Zugangsbereich zu der Tür 2 ein Sperrorgan, z. B. nach Art eines Drehkreuzes, angebracht sein, das einerseits den Eintritt immer erlaubt, andererseits aber das Verlassen des Waschraums nur dann, wenn zuvor die Tür 2 von innen, d. h. nach Freigabe der Verriegelungseinrichtung 8 geöffnet worden ist. Auf diese Weise kann verhindert werden, dass jemand den Waschraum unkontrolliert verlässt, nachdem die Tür 2 von einer anderen Person von außen geöffnet worden ist.

Denselben erhöhten Sicherheitsgrad erreicht man grundsätzlich mit einem Absperrorgan, z. B. in Form einer Tür, einer Klappe eines Schiebers oder eines Drehkreuzes, das Personen jeweils nur einzeln nacheinander passieren lässt und in der einen Bewegungsrichtung durch die Detektoreinheit 9 steuerbar ist, oder mit getrennten Absperrorganen, die einen Eingang und einen Ausgang jeweils zu Einbahnstraßen machen, wobei das Absperrorgan im Ausgang in der vorstehend beschriebenen Weise mit der Detektoreinheit 9 zusammenwirkt.

Schließlich könnte durch eine geeignete Zeitsteuerung und/oder Umgrenzung der Tür 2 und der Detektoreinheit 9 auf der Waschraumseite, z. B. auch mittels Lichtschranken, dafür gesorgt werden, dass jeweils nur diejenige Person den Raum verlassen kann, deren Hände unmittelbar zuvor von der Detektoreinheit geprüft worden sind. Damit wird verhindert, dass jemand mit gereinigten Händen eine andere Person vor oder nach sich durch die von ihm entriegelte Tür gehen lässt.

## Patentansprüche

1. Vorrichtung zur Desinfektionskontrolle, **dadurch gekennzeichnet, dass** an einem Ausgang eines Waschraums mit Einrichtungen zum Waschen und Desinfizieren der Hände mittels eines Desinfektionsmediums, das detektionsfähig ist oder eine detektionsfähige Markierungssubstanz enthält, ein verriegelbares Absperrorgan (2) angeordnet ist, dessen Verriegelungseinrichtung (8) von einer Detektoreinheit (9) steuerbar ist, die das Vorhandensein von Rückständen des Desinfektionsmediums bzw. der Markierungssubstanz an den Händen der Personen registriert, die den Waschraum verlassen wollen.

2. Vorrichtung nach Anspruch 1, **dadurch gekennzeichnet, dass** das Absperrorgan (2) derart angeordnet, ausgebildet und gesteuert ist, dass es jeweils nur den Durchtritt derjenigen Person nach außen zulässt, deren Handflächen von der Detektoreinheit (9) als desinfiziert erkannt worden sind.

3. Vorrichtung nach Anspruch 1 oder 2, **dadurch gekennzeichnet, dass** die Markierungssubstanz ein Fluoressenzfarbstoff ist, der durch eine an der Detektoreinheit (9) angebrachte Strahlungsquelle zur Fluoreszenzstrahlung anregbar ist und dessen emittierte Fluoreszenzstrahlung durch einen in der Detektoreinheit (9) enthaltenen Sensor (10) registrierbar ist.

4. Vorrichtung nach Anspruch 3, **dadurch gekennzeichnet, dass** dem Sensor (10) ein für die Fluoreszenzstrahlung durchlässiges Filter vorgeschaltet ist.

5. Vorrichtung nach einem der Ansprüche 1 bis 4, **dadurch gekennzeichnet, dass** die Detektoreinheit (9) einen im Infrarotbereich (800-1300nm) empfindlichen Sensor hat.

6. Vorrichtung zur Desinfektionskontrolle nach einem der Ansprüche 1 bis 5, **dadurch gekennzeichnet, dass** die Detektoreinheit (9) elektrisch mit der Verriegelungseinrichtung (8) gekoppelt ist.

7. Vorrichtung zur Desinfektionskontrolle nach einem der Ansprüche 1 bis 6, **dadurch gekennzeichnet, dass** um die Sensorfläche (10) der Detektoreinheit (9) ringförmig ein Kranz von Distanzstiften (18) vorgesehen ist.

8. Vorrichtung zur Desinfektionskontrolle nach Anspruch 7, **dadurch gekennzeichnet, dass** die Distanzstifte (18) eine Länge von 20 - 25 mm haben.
